# EUROPEAN PATENT SPECIFICATION

(11) **EP 3 733 110 B1**
(45) Date of publication and mention of the grant of the patent: **23.08.2023**
(21) Application number: 18896067.8
(22) Date of filing: 19.12.2018
(51) Int. Cl.: A61B 34/30, B25J 9/10, B25J 18/00, B25J 9/16, B25J 9/04, B25J 9/00, A61B 90/50

(54) **SURGICAL ROBOT TERMINAL**
CHIRURGISCHER ROBOTERANSCHLUSS
TERMINAL DE ROBOT CHIRURGICAL

(30) Priority: 27.12.2017 CN 201711449375
(43) Date of publication of application: 04.11.2020
(73) Proprietor: Shanghai Microport Medbot (Group) Co., Ltd., Shanghai 201203 (CN)
(72) Inventor: LI, Tao, Shanghai 201203 (CN); HE, Chao, Shanghai 201203 (CN); ZHANG, Yijia, Shanghai 201203 (CN); ZHOU, Bin, Shanghai 201203 (CN)
(74) Representative: Patentship Patentanwaltsgesellschaft mbH
(86) International application number: PCT/CN2018/122087
(87) International publication number: WO 2019/128803

(56) References cited:
- WO-A1-2006/079108
- WO-A2-2010/068005
- WO-A2-2010/068005
- CN-A- 102 973 317
- CN-A- 106 236 276
- CN-A- 106 618 736
- CN-A- 107 072 725
- CN-A- 107 374 736
- CN-A- 108 186 120
- CN-U- 206 151 581
- JP-A- 2012 005 557
- US-A1- 2004 261 179
- US-A1- 2016 374 771
- US-A1- 2017 079 722

## Description

### TECHNICAL FIELD

The present application relates to the technical field of medical devices and, more specifically, to a surgical robot terminal.

### BACKGROUND

The comprehensive development of robotics has brought about great advancements in the field of medical technology. A wide variety of robots with different structures for various uses, from the earliest AESOP^{®} laparoscope holder to the modem DA VINCI^{®} surgical robot, are applied to medical field.

As an advanced technology in the field of medical treatment, minimally invasive surgery is increasingly replacing the traditional open surgery. More and more patients are choosing minimally invasive surgery as their first choice for treatment because of its advantages including smaller surgical incisions, rapid recovery and good outcomes. Among various minimally invasive surgeries, the development of minimally invasive laparoscopic surgery is most outstanding from its inception almost at the same time as the advent of laparoscopy. Up to the date, it has been an established technique and is still developing, for example, toward the direction of combining with robotics to further facilitate surgeons' operations.

Most existing minimally invasive laparoscopic surgical robots are of a master-slave type, in which a surgeon manipulates two controllers at a master console to remotely control the robot's terminal incorporating several robotic arms on each of which a surgical instrument is mounted that is insertable into a patient to perform a desired operation. Whether a surgical operation can be smoothly carried out directly depends on the positions and orientations of the robotic arms, preoperative adaptations of the terminal to the operation are thus necessary before a robot-assisted surgical operation is performed.

In terms of allowing such adaptations, current surgical robots' terminals are found with the following issues:
(1) Limited Adjustability of Robotic Arms - Pat. Pub. No. CN104717936A proposes a terminal of a surgical robot, comprising a suspension disc arranged above robotic arms in order to adjust the overall robotic arms to an orientation suitable for access to a target surgical site. However, as pointed out in the publication itself, the robotic arms are limited in position relative to a cart in order to avoid tip-over due to shift in center of gravity. Additionally, the robotic arms are coupled to an orienting platform by rotatable joints and include respective base joints that are fixed with respect to one another. This imposes great limitations on an adjustment range.
(2) Lack of Utilization of Surgical Bed's Functionality - There are few current robots that utilize a surgical bed. A conventional robot is usually directly fixed to the floor or to a surgical bed on which a patient is lying, without any collaboration therewith, despite the fact that preliminary surgical preparation often requires the surgical bed to move in coordination with the robot to facilitate access of the latter to a target surgical site.

Pat. Pub. No. CN106456263A proposes a method and device for tele-surgical table registration. The surgical table (i.e., surgical bed) has a plurality of translational and rotational degrees of freedom as an extension of functionality, but as described in the publication, the table is physically arranged and controlled separately from a surgical robot, without an effective correlation mechanism established between them, resulting in high registration complexity and low registration reliability.

US 2017/079722 A1 discloses methods and systems for registering a manipulator assembly and independently positionable surgical table, wherein the methods include reading a fiducial marker on the surgical table with a sensor associated with the manipulator assembly and localizing the manipulator assembly and surgical table with respect to a common reference frame, wherein the methods further include translating a 3D configuration of the surgical table to a 2D frame of reference so as to estimate a 3D pose of the surgical table relative the manipulator assembly for use in coordinating movements therebetween. CN 107 374 736 A discloses a surgical cart comprising a standing column, wherein vertical guide rails are arranged on the outer walls of the standing column and a slider is slidably arranged on each vertical guide rail, wherein a base for mounting a mechanical arm is fixedly arranged on each slider, and limit devices for controlling the corresponding base to move in stroke limit are respectively arranged at the top and the bottom of each outer wall of the standing column. CN 106 236 276 A discloses a surgical robot system comprising robotic arms, a base, a support connected to the base, and a suspension structure connected to the support, wherein the suspension structure is connected to the robotic arms. WO 2006/079108 A1 discloses a robotic surgical system comprising a manipulator support assembly, wherein the manipulator support assembly comprises an orienting platform and a plurality of configurable set-up joint arms couplable to the orienting platform. US 2004/261179 A1 discloses a linkage structure rotatably connected to the ceiling. CN 106 618 736 A discloses a robotic arm comprising a support structure that functions as a support for the robotic arm. US 2016/374771 A1 discloses a robotic arm including a first actuating mechanism, a first arm segment, a second actuating mechanism, a second arm segment, a passive actuating mechanism, an active actuating mechanism, and a tool attachment interface. WO 2010/068005 A2 discloses a surgical robot comprising a main unit, a support arm joined to the main unit, and a robot arm joined to the support arm, wherein the support arm is composed of the major support arm, which may be joined to the main unit, and the minor support arm, which may be joined to an end portion of the major support arm.

### SUMMARY OF THE INVENTION

The invention is set out in the appended set of claims.

It is an objective of the some embodiments to provide terminal of a surgical robot to solve at least one of the problems existing in the conventional robots.

To this end, the present application provides a surgical robot according to the appended independent claim.

Optionally, in the surgical robot terminal, the axes of rotation of the first, second and third rotatable joints intersect one another at a single point.

Optionally, in the surgical robot terminal, the vertical assembly is located on one side of the surgical bed and fixedly connected to the base.

Optionally, in the surgical robot terminal, the surgical bed comprises a bed body and a support that is connected to the bed body and fixedly connected to the base, and
wherein the first, second and third rotatable joints are all arranged on the bed body.

Optionally, in the surgical robot terminal, the bed body comprises an outer frame and a hook joint disposed inside and connected to the outer frame, wherein the first and second rotatable joints are both arranged on the hook joint, and wherein the outer frame is connected to the support via the third rotatable joint.

Optionally, in the surgical robot terminal, the bed body comprises an outer frame and a hook joint disposed inside and connected to the outer frame, wherein the first, second and third rotatable joints are all arranged on the hook joint, and wherein the outer frame is fixedly connected to both the vertical assembly and the support.

Optionally, in the surgical robot terminal, the terminal further comprises:
a console configured to control operation of the base, the surgical bed, the vertical assembly and/or the robotic arm.

Optionally, in the surgical robot terminal, the support structure comprises
a primary arm support and at least two secondary arm supports;
wherein the primary arm support has an upper end rotatably connected to the support end of the vertical assembly; and
each of the at least two secondary arm supports is rotatably connected to a lower end of the primary arm support and has a lower end connected to the setup arm.

Optionally, in the surgical robot terminal, the support structure comprises:
at least one arm support, each having an upper end rotatably connected to the support end of the vertical assembly and a lower end connected to the setup arm.

Optionally, in the surgical robot terminal, the setup arm comprises a first rotary joint, a first horizontal translational joint, a swinging joint and a second rotary joint, which are sequentially connected together;
wherein the first horizontal translational joint has an axis of translation that is perpendicular to an axis of rotation of the first rotary joint,
the swinging joint has an axis of rotation that is perpendicular to both the axis of translation of the first horizontal translational joint and the axis of rotation of the first rotary joint,
the second rotary joint has an axis of rotation oriented parallel to the axis of rotation of the first rotary joint,
the first rotary joint connects the setup arm to the support structure, the second rotary joint rotatably connects the setup arm to the tool arm.

Optionally, in the surgical robot terminal, the setup arm further comprises a second horizontal translational joint having a proximal end connected to the support structure, a distal end connected to the first rotary joint and an axis of translation that is perpendicular to the axis of rotation of the first rotary joint.

According to the invention, in the surgical robot terminal, the setup arm comprises a first horizontal translational joint, a first rotary joint, a swinging joint and a second rotary joint, which are sequentially connected together;
wherein the first horizontal translational joint has an axis of translation that is perpendicular to an axis of rotation of the first rotary joint,
the swinging joint has an axis of rotation that is perpendicular to both the axis of translation of the first horizontal translational joint and the axis of rotation of the first rotary joint,
the second rotary joint has an axis of rotation oriented parallel to the axis of rotation of the first rotary joint,
the first horizontal translational joint connects the setup arm to the support structure, the second rotary joint rotatably connects the setup arm to the tool arm.

Optionally, in the surgical robot terminal, the setup arm comprises a first rotary joint, a first horizontal translational joint, a swinging joint and a second rotary joint, which are sequentially connected together;
wherein the first horizontal translational joint has an axis of translation that is perpendicular to an axis of rotation of the first rotary joint,
the swinging joint has an axis of rotation that is perpendicular to both the axis of translation of the first horizontal translational joint and the axis of rotation of the first rotary joint,
the second rotary joint has an axis of rotation oriented parallel to the axis of rotation of the first rotary joint,
the first rotary joint connects the setup arm to the vertical assembly, the second rotary joint rotatably connects the setup arm to the tool arm.

Optionally, in the surgical robot terminal, the swinging joint comprises a rotatable first parallelogram structure comprising a first proximal link and a first distal link parallel to the first proximal link, the first proximal link has an axis that is parallel to or collinear with the axis of rotation of the first rotary joint, the first distal link is connected to the second rotary joint, the first distal link has an axis that is parallel to or collinear with the axis of rotation of the second rotary joint.

Optionally, in the surgical robot terminal, the setup arm further comprises a connecting rod rotatably connected to both the swinging joint and the second rotary joint, a measurement component configured to measure a swing angle of the swinging joint and a motor configured to drive the second rotary joint to swing relative to the connecting rod;
wherein the measurement component is communicatively connected to the motor configured to drive the second rotary joint to swing in accordance with the swing angle of the swinging joint received from the measurement component so that the axis of rotation of the second rotary joint is maintained parallel to the axis of rotation of the first rotary joint.

Optionally, in the surgical robot terminal, tool arm comprises a base joint, a rotatable second parallelogram structure and a telescopic joint;
wherein the base joint is configured to pivot about a first axis so as to drive the surgical instrument to pivot about the first axis, the base joint has a proximal end connected to the setup arm and a distal end connected to the second parallelogram structure,
the second parallelogram structure comprising a second proximal link and a second distal link parallel to the second proximal link, the second parallelogram structure is configured to drive the surgical instrument to swing about a second axis,
the telescopic joint connected to the second distal link, the telescopic joint has an axis of translation that is parallel to an axis of the second distal link, the telescopic joint is detachably connected to the surgical instrument so as to be able to drive the surgical instrument to translate along the axis of translation of the telescopic joint, and
the remote center of motion is defined at an intersection of the first axis, the second axis and the axis of translation of the telescopic joint.

Optionally, in the surgical robot terminal, the tool arm comprises a base joint and a rotatable third parallelogram structure;
wherein the base joint is configured to pivot about a first axis so as to drive the surgical instrument to pivot about the first axis, the base joint has a proximal end connected to the setup arm and a distal end connected to the third parallelogram structure,
the third parallelogram structure comprises a third proximal link and a third distal link parallel to the third proximal link, the third parallelogram structure having a terminal end connected to the surgical instrument having an axis parallel to an axis of the distal link so that the third parallelogram structure is able to drive the surgical instrument to pivot about a second axis, and
the remote center of motion is defined at an intersection of the first axis and the second axis.

Optionally, in the surgical robot terminal, the vertical assembly further comprises a horizontal translational joint that is disposed proximally at the support end of the vertical assembly and configured to adjust a position of the remote center of motion along a translation direction of the horizontal translational joint.

Optionally, in the surgical robot terminal, the terminal further comprises:
a plurality of universal wheel disposed at a bottom of the base in order to change a position of the base relative to the surgical bed.

In the surgical robot terminal provided in the present application, the support structure rotatably coupled at the upper end to the support end of the vertical assembly and coupled at the lower end to each setup arm allows easy, fine adjustments to the position of the RC in various operation environments, and thus a better adaption to various operation environment. Additionally, the surgical bed's design with multiple rotary joints allows it to be adjusted relative to the orientation of the robotic arms so as to enable each robotic arm to operate in a space more suitable for a higher degree of freedom in pivoting about the RC. The adjustability of the surgical bed relative to the robotic arms compensates for the robotic arms' limited movement range. As a result, the surgical robot terminal can reach any desired working position simply by coordinated movement of the rotary joints on the surgical bed. This allows a simpler overall structure of the terminal with a reduced weight, making it more consistent with the demand for lightweight, structurally simpler medical robots.

Further, the vertical assembly and the base can cooperate with each other to allow each robotic arm in the terminal to get closer to the RC so that it can be located at a more suitable position that can facilitate the implementation of a surgical operation.

Furthermore, adjusting the robotic arms' spatial positions using the arm support(s) can effectively avoid interference between these robotic arms when they are moving, achieving better adjustment results.

### BRIEF DESCRIPTION OF THE DRAWINGS

Fig. 1 is a principal view of a surgical robot terminal according to Embodiment 1 of the present application.
Fig. 2 schematically illustrates a degree-of-freedom distribution of a surgical bed shown in Fig. 1.
Fig. 3 schematically illustrates the structure of one example of the surgical robot terminal according to Embodiment 1 of the present application in which it incorporates one arm support.
Fig. 4 schematically illustrates the structure of another example of the surgical robot terminal according to Embodiment 1 of the present application in which it incorporates one primary arm support and two secondary arm supports.
Fig. 5 is a principal view of a surgical robot terminal according to Embodiment 2 of the present application.
Fig. 6 schematically illustrates the structure of one example of the surgical robot terminal according to Embodiment 2 of the present application in which it incorporates one primary arm support and two secondary arm supports.
Fig. 7 is a principal view of a surgical robot terminal according to Embodiment 3 of the present application.

In these figures, 1 denotes a base; 2 denotes a vertical assembly; 21 denotes a vertical translational joint; 22 denotes a horizontal translational joint; 200 denotes a support end; 201 denotes a base end; 3 denotes a robotic arm; 31 denotes a setup arm; 311 denotes a first horizontal translational joint; 312 denotes a swinging joint; 313 denotes a second rotary joint; 314 denotes a first rotary joint; 32 denotes a tool arm; 321 denotes a base joint; 322 denotes a parallelogram structure; 323 denotes a telescopic joint; 4 denotes a surgical bed; 41 denotes a bed body; 410 denotes a first rotatable joint; 411 denotes a second rotatable joint; 42 denotes support; 420 denotes a third rotatable joint; 5 denotes a console; 7 denotes a universal wheel; 8 denotes an arm support; 9 denotes a primary arm support; 10 denotes a secondary arm support; and RC denotes a remote center of motion (RCM).

### DETAILED DESCRIPTION

Specific embodiments of surgical robot terminal according to the present application will be described in greater detail below with reference to the accompanying drawings. Features and advantages of the application will be more apparent from the following detailed description, and from the appended claims. Note that the figures are provided in a very simplified form not necessarily presented to scale, with their only intention to facilitate convenience and clarity in explaining the embodiments.

Throughout this specification, a "proximal end" of an article is meant to refer to an end thereof located closer to a vertical assembly, while a "distal end" or "terminal end" is meant to refer to an end farther away from the vertical assembly.

### Embodiment 1

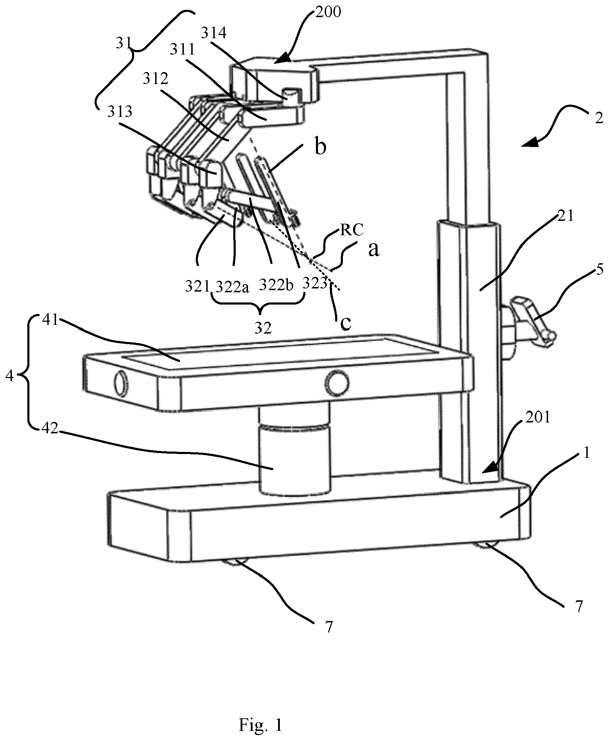

Referring to Fig. 1, a surgical robot terminal according to the present application essentially includes a base 1, a vertical assembly 2, at least one robotic arm 3 and a surgical bed 4. As shown in Fig. 1, the terminal includes the base 1, the vertical assembly 2, four robotic arms 3 and the surgical bed 4. Each robotic arm 3 includes a setup arm 31 and a tool arm 32. The tool arm 32 is coupled to a surgical instrument and configured to drive the surgical instrument to pivot about a remote center (RC) of motion, and a distal end of the setup arm 31 is coupled to the tool arm 32. Preferably, the distal end of the setup arm 31 is rotatably coupled to the tool arm 32. The setup arm 31 is configured to adjust a spatial position of the RC. The surgical bed 4 is connected to the base 1 and includes a first rotatable joint 410, a second rotatable joint 411 and a third rotatable joint 420. The first, second and third rotatable joints 410, 411, 420 have respective axes of rotation, which are so arranged that any of them is perpendicular to both the other two. Preferably, the axes of rotation of the first, second and third rotatable joints 410, 411, 420 are perpendicular to one another and intersect one another at a single point so as to allow the surgical bed 4 to be adjusted in orientation relative to the RC. The vertical assembly 2 remains stationary with respect to the surgical bed 4 and has a support end 200 located above the surgical bed 4. The vertical assembly 2 includes a vertical translational joint 21 configured to adjust a height of the RC relative to the base 1. A proximal end of the setup arm 31 is coupled to the support end 200 of the vertical assembly 2. Preferably, the terminal further includes a support structure having an upper end rotatably coupled to the support end 200 and a lower end coupled to the setup arm 31. A target surgical site in a patient (i.e., a target position for the RC) is taken as a reference point for positional adjustment of the terminal, and according to the present application, the support structure allows the RC to be easily and finely tuned to the target position (i.e., the patient's target surgical site) in various surgical environments while effectively avoiding interference between the setup arms. In addition, the surgical bed 4 can be adjusted in orientation to assist each setup arm to tune the RC to the target position. This can facilitate the implementation of various surgical operations requiring different positions and orientations of the robotic arms 3.

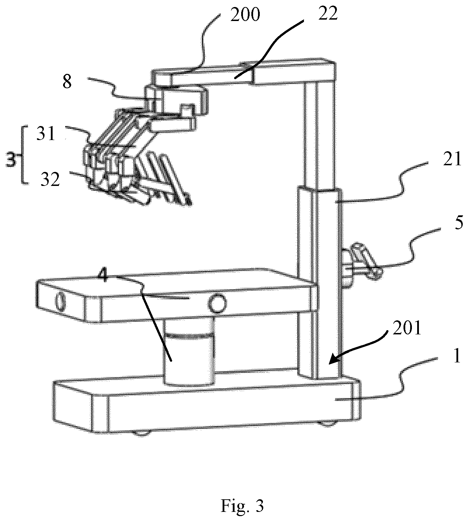

For example, in the case that an orientation of the surgical bed 4 set initially is perpendicular to a horizontal portion or horizontal translational joint 22 (detailed below in conjunction with Fig. 3) of the vertical assembly 2, if a surgical instrument is required to insert into an incision made right above the patient's abdomen toward the head of the patient, the support structure must rotate by 90° to meet the operation requirement when only the robotic arms 3 are adjustable. By contrast, as the surgical bed 4 also allows adjustments according to the present application, the task can be fulfilled by collaboratively rotating both the support structure and the surgical bed. For example, in the case that an incision is made lateral to the patient's abdomen, it is necessary for the patient to lie sideways in surgical bed and for the support structure to be adjusted with an angle. By contrast, both the surgical instrument and the surgical bed can be adjusted in coordination to make more suitable work space for surgical instrument allowing it to operate more easily.

With continued reference to Fig. 1, in this application, the vertical assembly 2 is arranged at a side of the surgical bed 4 and is fixedly coupled to the base 1.

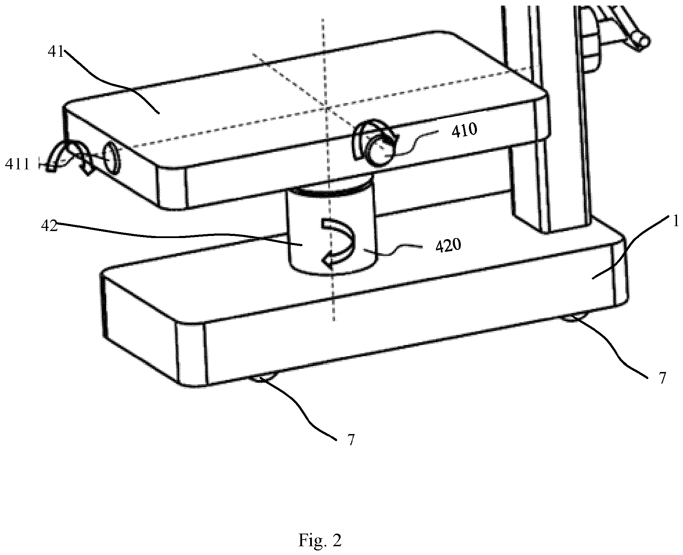

Referring to Figs. 1 and 2, the surgical bed 4 includes a bed body 41 and a support 42 that is connected to the bed body 41 and secured to the base 1. As shown in Fig. 1, the support 42 is connected to the bed body 41 at one end and secured to the base 1 at the other end. That is, the base 1 serves as a carrier for both the surgical bed 4 and the vertical assembly 2. The first, second and third rotatable joints 410, 411, 420 are arranged on the bed body 41. In a particular embodiment, the bed body 41 includes an outer frame and a hook joint that is disposed inside and connected to the outer frame. In this case, the first and second rotatable joints 410, 411 are arranged on the hook joint, and the outer frame is connected to the support 42 by the third rotatable joint 420. For example, the hook joint includes: a first rotatable member that is disposed inside the outer frame and rotatably coupled thereto by the first rotatable joint 410; and a second rotatable member that is arranged within the first rotatable member and rotatably coupled thereto by the second rotatable joint 411. Here, the first and second rotatable joints 410, 411 impart first and second degrees of freedom to the hook joint. The outer frame is rotatably coupled to the support 42 via the third rotatable joint 420, achieving a third degree of freedom. The bed body 41 can move under the action of the first, second and third rotatable joint 410, 411, 420 to change its orientation relative to the RC. As such, in scenarios where preliminary preparation for a surgical operation would conventionally be considered difficult to carry out, such as when it is required to pivot the robotic arms 3 over a large angle or place the patient on his/her side or in another position in order to allow a surgical instrument to access a target surgical site, the bed body 41 can be rotated to a suitable position where the surgical instrument can more easily access the surgical site without requiring a large stroke of the robotic arms 3 or a change in the patient's position, thus facilitating the implementation of the surgical operation.

In order to facilitate positional adjustment of the surgical robot terminal, the surgical robot terminal may further include a plurality of universal wheels 7 that are provided at the bottom of the base 1 in order to allow changes in the position of the base 1.

Further, referring to Figs. 1 and 3, the vertical assembly 2 generally resembles the inverted letter "L". Specifically, in the embodiment of Fig. 1, the vertical assembly 2 includes a vertical translational joint 21, a horizontal beam and a vertical column fixedly connected to a proximal end of the horizontal beam. The support end 202 forms a distal end of the horizontal beam. The vertical translational joint 21 is arranged on the vertical column (corresponding to a longer side of the L-like shape) and configured to vertically displace the robotic arms 3. In another embodiment shown in Fig. 3, the vertical assembly 2 includes a vertical translational joint 21, a horizontal translational joint 22, a horizontal beam and a vertical column fixedly connected to a proximal end of the horizontal beam. The horizontal translational joint 22 is located proximally at the support end of the vertical assembly. Specifically, the vertical translational joint 21 is arranged on the vertical column (corresponding to the longer side of the L-like shape), while the horizontal translational joint 22 is arranged on the horizontal beam (corresponding to a shorter side of the L-like shape). The vertical translational joint 21 is configured to allow the vertical assembly 2 to move along the direction, in which the vertical translational joint is moveable, thus adjusting a position of the RC with respect to the same direction. The horizontal translational joint 22 arranged at the support end of the vertical assembly is configured to adjust a position of the RC with respect to the direction in which the horizontal translational joint 22 is movable.

Each setup arm 31 configured as steric configuration with at least four degrees of freedom includes two rotational degrees of freedom (embodied in first and second rotary joints 314, 313 in the embodiment of Fig. 1), a horizontal translational degree of freedom (embodied in first horizontal translational joints 311 in the embodiment of Fig. 1) and a swinging degree of freedom (embodied in swinging joints 312 in the embodiment of Fig. 1). In the embodiment of Fig. 1, each setup arm 31 includes a first rotary joint 314, a first horizontal translational joint 311, a swinging joint 312 and a second rotary joint 313, which are sequentially connected together in this order, the first horizontal translational joint 311 having an axis of translation (i.e., a movement trajectory or a telescopic direction thereof, or a track in case of the joint being implemented as a slider-track structure) that is perpendicular to an axis of rotation of the first rotary joint 314, the swinging joint 312 having an axis of rotation that is perpendicular to both the axis of translation of the first horizontal translational joint 311 and an axis of rotation of the first rotary joint 314, the second rotary joint 313 having an axis of rotation oriented parallel to the axis of rotation of the first rotary joint 314. The first rotary joint 314 couples the setup arm 31 to the vertical assembly 2, and the second rotary joint 313 rotatably couples the setup arm 31 to the tool arm 32. The first rotary joint 314 is able to drive the whole robotic arm 3 to rotate about the axis of rotation of the first rotary joint 314, and the first horizontal translational joint 311 can drive the tool arm 32 to translate horizontally. The swinging joint 312 is configured to swing so as to drive the tool arm 32 to move within a vertical plane. The first and second rotary joints 314, 313 are redundant to each other and configured to enable finer adjustments. These four joints of the setup arm 31 can work together to achieve a change of the RC in spatial position.

The present embodiment is not limited to any particular method for making the axis of rotation of the second rotary joint 313 parallel to that of the first rotary joint 314. In a preferred embodiment, the setup arm further includes a connecting rod, a measurement component and a motor communicatively coupled to the measurement component. A proximal end of the connecting rod is coupled to the swinging joint 312, and a distal end thereof is rotatably coupled to the second rotary joint 313. The measurement component is configured to measure a swing angle of the swinging joint 312 in real time. The motor is configured to drive the second rotary joint 313 to rotate relative to the connecting rod in accordance with the swing angle of the swinging joint 312 so as to always maintain the axis of rotation of the second rotary joint 313 parallel to that of the first rotary joint 314. In another preferred embodiment, the swinging joint 312 includes a rotatable first parallelogram structure (e.g., formed by four links articulated together) including a first proximal link and a distal link that is parallel to the first proximal link. An axis of the first proximal link in the first parallelogram structure is parallel to or collinear with the axis of rotation of the first rotary joint 314, while the first distal link (that is parallel to the first proximal link) in the first parallelogram structure is coupled to the second rotary joint 313 and has an axis parallel to or collinear with the axis of rotation of the second rotary joint 313. In this way, the axis of rotation of the second rotary joint 313 is always maintained parallel to that of the first rotary joint 314.

The tool arm 32 is an RC mechanism capable of driving the surgical instrument coupled thereto to pivot about a remote center (RC). For example, the tool arm 32 may have multiple degrees of freedom, for example, two degrees of freedom (in this case, the tool arm 32 is able to drive the surgical instrument to swing about the RC in both left-and-right and front-and-back directions) or three degrees of freedom (in this case, the tool arm 32 is able to drive the surgical instrument to swing about the RC in both left-and-right and front-and-back directions and move up and down).

In the embodiment of Fig. 1, the tool arm 32 has three degrees of freedom so as to be able to drive the surgical instrument to pivot about a first axis "a", swing about a second axis "c" and translate along an axis of translation "b", and the RC is defined at the intersection of the first axis "a", axis of translation "b" and second axis "c" (the three axes are indicated by dashed lines in the figure). Specifically, the tool arm 32 includes a base joint 321, a rotatable second parallelogram structure (e.g., formed by four links articulated together) and a telescopic joint 323. The base joint 321 is configured to rotate about the first axis "a" so as to drive the surgical instrument to swing about the first axis "a". A proximal end of the base joint 321 is coupled to the setup arm 31, and a distal end thereof is coupled to the second parallelogram structure. The second parallelogram structure includes a second proximal link and a second distal link parallel to the second proximal link. A terminal end of the second parallelogram structure is coupled to the surgical instrument through the telescopic joint 323, and the surgical instrument is configured such that an axis thereof is parallel to an axis of the second distal link. As a result, the second parallelogram structure is able to drive the surgical instrument to pivot about the second axis "c". In this embodiment, the second parallelogram structure includes a first parallelogram sub-structure 322a and a second parallelogram sub-structure 322b connected thereto. That is, the second parallelogram structure for driving the surgical instrument to swing about the second axis "c" forms a double-parallelogram structure. The first parallelogram sub-structure 322a includes a first proximal sub-link and a first distal sub-link (not shown) parallel to the first proximal sub-link. Similarly, the second parallelogram sub-structure 322b includes a second proximal sub-link and a second distal sub-link (not shown) parallel thereto. An axis of the first distal sub-link coincides with or extends parallel to that of the second proximal or distal sub-link. The second parallelogram sub-structure 322b has a terminal end coupled to the telescopic joint 323, which defines the axis of translation "b" that is parallel to the axis of the second distal sub-link in the second parallelogram sub-structure 322b and extends through the RC. The telescopic joint 323 is detachably coupled to the surgical instrument so as to allow the surgical instrument to translate along the axis of translation "b".

In an alternative embodiment, the tool arm 32 may have only two degrees of freedom so as to be able to drive the surgical instrument to pivot about the first axis "a" and swing about the second axis "c", with the RC being defined at the intersection of the first and second axes "a", "c". In this case, the tool arm 32 includes a base joint 321 and a rotatable third parallelogram structure (e.g., formed by four links articulated together) including a third proximal link and a third distal link parallel to the third proximal link. A terminal end of the third parallelogram structure is detachably coupled to the surgical instrument, and the axis of the surgical instrument is parallel to an axis of the distal link in the third parallelogram structure. With this design, the third parallelogram structure is able to drive the surgical instrument to swing about the second axis "c". Further, the axis of the surgical instrument extends through the RC. It will be appreciated by those skilled in the art that the tool arm may adopt another structure for driving the surgical instrument to swing about the RC, such as a structure incorporating a base joint and a slider-arcuate track structure coupled to the base joint.

During preliminary preparation for a surgical operation, according to the present embodiment, as a result of movement of the various joints, the setup arm 31 drives the RC of the tool arm 32 to approach a target surgical site in the patient, thereby facilitating the implementation of a more accurate surgical operation. In general terms, prior to a surgical operation is performed, the RC is brought to the vicinity of the patient's target surgical site by suitably moving the various joints of the setup arm 31. During the surgical operation, all the joints of the setup arm 31 are locked so that the RC is fixed in position, and the surgical instrument detachably coupled to the tool arm 32 is configured to insert into the patient via an incision around the RC to directly access the target surgical site. The surgical instrument can be driven by the tool arm 32 to swing and pivot within a conical working space with an apex located at the RC. In the course of the surgical operation, if there emerges a need for an additional adjustment to the position of the RC, one or more appropriate joints in the setup arm 31 may be released from locking, or the orientation of the bed body 41 may be changed, in order to accomplish the required adjustment. Therefore, adjusting the RCM's position through the setup arm 31 allows the surgical instrument mounted on the tool arm 32 to access the target surgical site, and adjusting the individual joints of the tool arm 32 enables the surgical instrument to move within a RC-defined space suitable for a desired particular surgical operation.

In order to ensure accurate control of the various components in the surgical robot terminal, the surgical robot terminal may include a console 5 for controlling operation of the base 1, the vertical assembly 2 and/or the robotic arms 3. For example, the console 5 may be located on the side of the vertical assembly 2 away from the surgical bed 4. Under the control of the console 5, the base 1 can move on the floor to deliver the surgical robot terminal to a position suitable for surgical operation; the vertical assembly 2 can move up or down to adjust the robotic arms 3 to a height relative to the surgical bed 4, which is more suitable for operation of the robotic arms; the first rotatable joint 410, the second rotatable joint 411 and/or the third rotatable joint 420 can move to tune the surgical bed 4 to a more reasonable orientation relative to the robotic arms' terminal ends while avoiding interference between the robotic arms 3 when they are moving during the surgical operation; and each setup arm 31 can move to make the RC get nearer to the target surgical site before the operation is started and to place the corresponding tool arm 302 at an optimal position for the implementation of the operation.

As shown in Figs. 1 and 3, the support structure includes at least one arm support 8 having an upper end rotatably coupled to the support end 200 of the vertical assembly 2 and a lower end coupled to the setup arms 31. The arm support 8 allows effective avoidance of interference between the robotic arms 3 when they are moving and can rotate to effectively change a spatial position of each setup arm 31 and thus facilitate positional adjustment by the setup arm 31 to the RC thereof. Specifically, the arm support 8 may be coupled to the support end 200 of the vertical assembly 2 via a rotatable joint so that when it rotates the overall orientation of the robotic arms 3 will change, allowing a wider reachable range thereof during surgery. Furthermore, the arm support 8 and the third rotatable joint 420 in the surgical bed 4 may rotate in coordination with each other in order to allow more accurate movement.

As shown in Fig. 3, the surgical robot terminal includes one arm support 8 and four robotic arms 3 all coupled to the arm support 8. In another preferred embodiment, two arm supports 8 may be included, to each of which two of the setup arms are coupled in such a manner that they are located on opposing sides of the arm support 8.

In one preferred embodiment, a second horizontal translational joint is additionally arranged between the setup arms 31 and the arm support 8. In this case, the first rotary joints 314 of the setup arms 31 are coupled to the arm support 8 via the second horizontal translational joint. This can result in an effectively expanded movement range of the robotic arms 3.

In another preferred embodiment, the four joints in each setup arm 31 are sequentially coupled together alternatively in the following order: the first horizontal translational joint 311, the first rotary joint 314, the swinging joint 312 and the second rotary joint 313. In this case, the setup arms 31 are coupled to the arm support 8 at their respective first horizontal translational joints 311. This arrangement allows full utilization of a space around the support end 200 of the vertical assembly 2 and the arm support 8.

In another embodiment, the support structure includes a primary arm support 9 and at least two secondary arm supports 10, the primary arm support 9 having an upper end rotatably coupled to the support end 200 of the vertical assembly 2, each of the at least two secondary arm supports 10 rotatably coupled to a lower end of the primary arm support 9 and having a lower end coupled to one or more setup arms 31. The primary arm support 9 can rotate to change spatial positions of the secondary arm supports 10, and in turn, each of the secondary arm supports 10 can rotate to change a spatial position of each setup arm 31 coupled thereto.

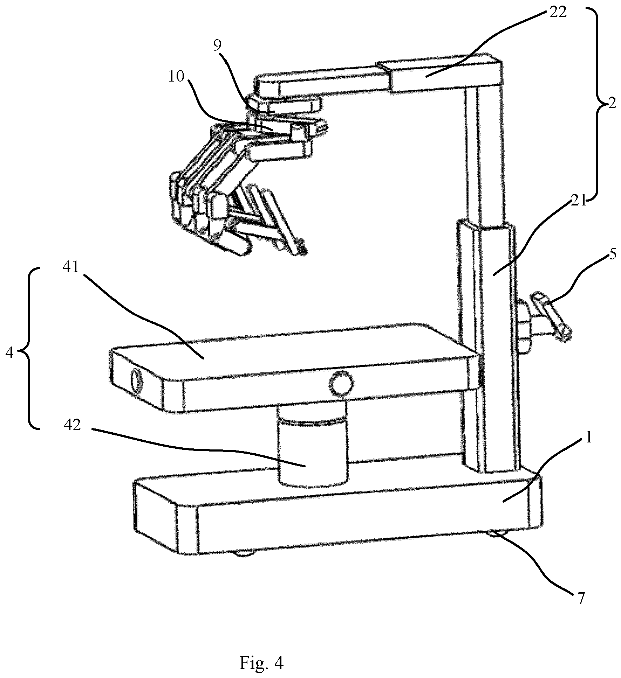

In the embodiment shown in Fig. 4, one primary arm support 9 and two secondary arm supports 10 are included. Each secondary arm support 10 has an upper end rotatably coupled to the primary arm support 9 so that it can rotate relative to the primary arm support 9. The primary arm support 9 is able to adjust a position of the secondary arm supports 10, which enables a rotation of the robotic arms 3 as a whole. The secondary arm supports 10 are able to adjust the setup arms 31 coupled thereto. Further, each secondary arm support 10 may be coupled at the lower end to two setup arms 31 in such a manner that the setup arms 31 are located at opposing sides of the specific secondary arm support 10. In other words, the secondary arm support 10 spaces the two setup arms 31 apart from each other. This can avoid interference between the setup arms 31 during use. The combination of the moveable primary arm support 9, secondary arm supports 10 and surgical bed 4 entails a redundant design allowing each surgical instrument to pivot about the RC. In this way, the surgical instrument can reach a position suitable for performing a surgical operation more accurately on a target surgical site.

In a preferred embodiment, a second horizontal translational joint is additionally arranged between the setup arms 31 and the secondary arm supports 10. In this case, each setup arm 31 is coupled, at its first rotary joint 314, to a corresponding one of the secondary arm supports 10 via the second horizontal translational joint. This can effectively expand the movement range of the robotic arms 3.

In another preferred embodiment, the four joints in each setup arm 31 are sequentially coupled together alternatively in the following order: the first horizontal translational joint 311, the first rotary joint 314, the swinging joint 312 and the second rotary joint 313. In this case, the setup arms 31 are coupled to the secondary arm supports 10 at their respective first horizontal translational joints 311. This arrangement allows full utilization of a space around the support end 200 of the vertical assembly 2 and the secondary arm supports 10.

In this configuration, the robotic arms 3 can be adjusted as a whole to a more suitable working position by adjusting the primary arm support 9. At the same time, the patient can be placed in an optimal position by adjusting the surgical bed 4. In addition, by adjusting the secondary arm supports 10, each robotic arm can reach a suitable position where interference between these robotic arms during surgery can be avoided. Rotating the secondary arm supports 10 in redundancy to rotating the surgical bed 4 can ensure optimal adjustment effects.

As can be seen from the above, the surgical robot terminal according to the present application enables each surgical instrument to reach a suitable position for a desire surgical operation by adjusting a spatial position of the corresponding setup arm using the arm supports 8, adjusting the RCM's position using the setup arm 31 and/or adjusting the surgical instrument's orientation relative to the RC using the tool arm 32. In addition, the patient can be placed in the most suitable position with a most suitable orientation by adjusting the surgical bed 4. Therefore, the surgical robot terminal can meet the current clinical need for improved surgical accuracy.

### Embodiment 2

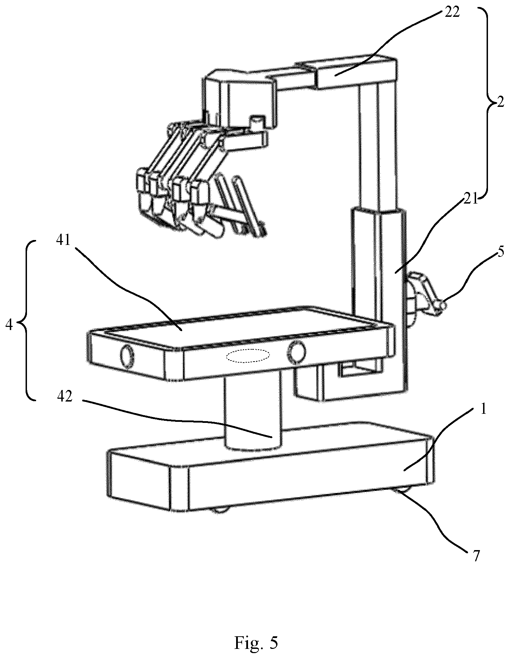

Reference is now made to Fig. 1, in conjunction with Fig. 5, a principal view of a surgical robot terminal according to the present application. From a comparison between the principal views of Figs. 1 and 5, it can be found that they show two distinct terminal configurations, which differ from each other essentially in the structure of the vertical assembly 2. Specifically, although the vertical assembly 2 is maintained at a fixed position relative to the surgical bed in both the embodiments of Figs. 1 and 5, this is accomplished in different manners. In the embodiment of Fig. 1, a base end 201 (i.e., the end of the vertical assembly opposite to the support end 200) of the vertical assembly 2 is fixedly connected to the base 1, while in the embodiment of Fig. 5, the base end 201 (i.e., the other end of the vertical assembly than the support end 200) of the vertical assembly 2 is fixedly connected to the surgical bed 4. In addition, although the bed body 41 also includes an outer frame and a hook joint disposed inside and coupled to the outer frame in this embodiment, the outer frame is fixedly connected to the support 42, and the vertical assembly 2 is secured to the outer frame. In this embodiment, in addition to the first and second rotatable joints 410, 411, the third rotatable joint 420 is also arranged on the hook joint. For example, in addition to the first rotatable member disposed inside the outer frame and the second rotatable member disposed within the first rotatable member, the hook joint may include a third rotatable member that is so arranged within the second rotatable member that it is rotatable relative to the second rotatable member by means of the third rotatable joint 420. Additionally, the second rotatable member is rotatably coupled to the first rotatable member through the second rotatable joint 411, and the first rotatable member is rotatably coupled to the outer frame by the first rotatable joint 410. As such, the hook joint incorporating the first, second and third rotatable joints 410, 411, 420 are able to rotate about axes of rotation of these three joints, i.e., having three degrees of freedom.

Similar to the above embodiment, in order to allow the robotic arms 3 to move in a wider horizontal range, in particular, to facilitate horizontal adjustment of the RC, a horizontal translational joint 22 is provided proximally at the support end 200 of the vertical assembly 2.

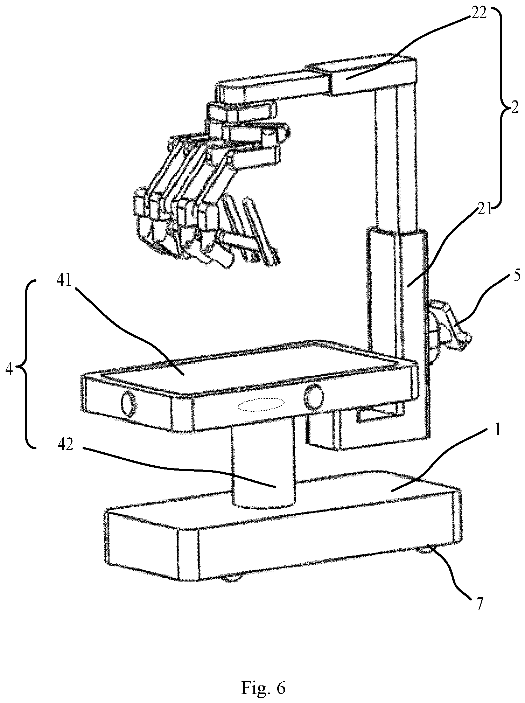

Reference is now made to Fig. 6, a schematic illustration of the surgical robot terminal additionally including a primary arm support 9 and two secondary arm supports 10, compared to the embodiment shown in Fig. 5. During the preparation for a surgical operation, under the control of the console 5, the primary arm support 9 moves to apply rough adjustments to spatial positions of the secondary arm supports 10, and the secondary arm supports 10 then rotate to change spatial positions of the setup arms 31 so that the robotic arms 3 reach respective optimal working positions. Alternatively, the robotic arms 3 may be brought to the optimal working positions by manually adjusting the robotic arms 3, the primary arm support 9 and the secondary arm supports 10. In the course of the surgical operation, the surgical bed 4 can cooperate with the vertical assembly 2, the primary arm support 9 and the secondary arm supports 10 to place the patient in a position with an orientation suitable for the implementation of the operation.

### Embodiment 3

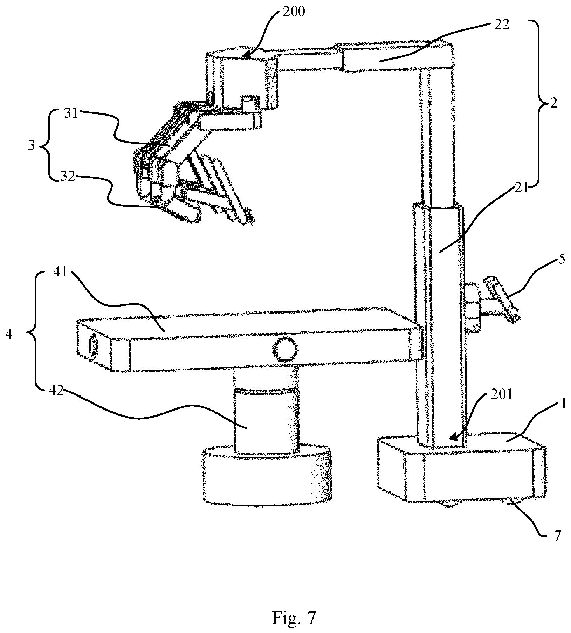

Reference is now made to Fig. 7, a principal view of a surgical robot terminal according to the present application, which has a configuration differing from those of Embodiments 1 and 2 in that the vertical assembly 2 is adjustable in position relative to (i.e., movable toward or away from) the surgical bed 4, while in Embodiments 1 and 2, the vertical assembly 2 is fixed (i.e., non-adjustable) in position relative to the surgical bed 4.

As shown in Fig. 7, the surgical robot terminal includes at least one robotic arm 3, a surgical bed 4, a base 1 and a vertical assembly 2. Each robotic arm 3 includes a setup arm 31 and a tool arm 32. The tool arm 32 is coupled to a surgical instrument and configured to drive the surgical instrument to pivot about an RC, and a distal end of the setup arm 31 is coupled to the tool arm 32. The setup arm 31 is configured to adjust a spatial position of the RC. The surgical bed 4 includes a first rotatable joint 410, a second rotatable joint 411 and a third rotatable joint 420. The first, second and third rotatable joints 410, 411, 420 have respective axes of rotation arranged to be perpendicular to one another. Preferably, the axes of rotation of the first, second and third rotatable joints 410, 411, 420 that are perpendicular to one another intersect one another at a single point so as to allow the surgical bed 4 to be adjusted in orientation relative to the RC. The base 1 is adjustable in position relative to the surgical bed 4. The vertical assembly 2 has a base end 201 fixedly connected to the base 1 (so that the vertical assembly 2 is adjustable in position under the action of the base 1) and a support end 200 located above the surgical bed 4. The vertical assembly 2 includes a vertical translational joint 21 configured to adjust a height of the RC relative to the base 1. A proximal end of the setup arm 31 is coupled to the support end 200 of the vertical assembly 2. Preferably, the proximal end of the setup arm 31 is rotatably coupled to the support end 200 of the vertical assembly 2.

In order to facilitate movement of the base 1, the surgical robot terminal may further include a plurality of universal wheels 7 that are provided at the bottom of the base 1 and configured to allow changes in the position of the base 1 relative to the surgical bed 4. As the base 1 is able to move freely, it can approach the patient at a suitable position compatible with conditions of the patient, and in other words, the RC also approaches the patient during the approaching of the base 1 to the patient. This greatly simplifies the structure of the support end 200 of the vertical assembly 2 and hence overall structure of the surgical robot terminal while reducing the total mass thereof, making it more consistent with the demand for lightweight, structurally simpler medical robots.

Similar to the above embodiments, in order to allow the robotic arms 3 to move in a wider horizontal range, in particular, to facilitate horizontal adjustment of the RC, a horizontal translational joint 22 is provided proximally at the support end 200 of the vertical assembly 2.

The embodiments disclosed herein are described in a progressive manner, with the description of each embodiment focusing on its differences from others. Reference can be made between the embodiments for their identical or similar parts.

In summary, in the surgical robot terminal provided in the present application, the support structure rotatably coupled at the upper end to the support end of the vertical assembly and coupled at the lower end to each setup arm allows easy, fine adjustments in the position of the RC in various surgical environments. Additionally, design of the surgical bed with multiple rotatable joints allows adjustments to the orientation thereof relative to the robotic arms so as to enable each robotic arm to operate in a more suitable space with a higher degree of freedom in pivoting about the RC. The adjustability of the surgical bed relative to the robotic arms compensates for the robotic arms' limited movement range. As a result, the surgical robot terminal can reach any desired working position simply by coordinated movement of the rotatable joints on the surgical bed. This allows a simpler overall structure of the surgical robot terminal with a reduced weight, making it more consistent with the demand for lightweight, structurally simpler medical robots.

Further, the vertical assembly and the base can cooperate with each other to allow each robotic arm in the surgical robot terminal to get closer to the RC so that it can be located at a more suitable position that can facilitate the implementation of a surgical operation.

Furthermore, adjusting the robotic arms' spatial positions using the arm support(s) can effectively avoid interference between these robotic arms when they are moving, achieving better adjustment results.

The description presented above is merely that of a few preferred embodiments of the present application and does not limit the scope thereof in any sense, which is defined by the attached independent claim.

## Claims

1. A surgical robot terminal, comprising:
a base (1);
at least one robotic arm (3), each comprising a setup arm (31) and a tool arm (32), the tool arm (32) being connected to a surgical instrument and configured to drive the surgical instrument to pivot about a remote center of motion, the setup arm (31) having a distal end connected to the tool arm (32), the setup arm (31) being configured to adjust a spatial position of the remote center of motion;
a surgical bed (4) comprising a first rotatable joint (410), a second rotatable joint (411) and a third rotatable joint (420), the first, second and third rotatable joints (410, 411, 420) having respective axes of rotation so arranged that any two thereof are perpendicular to each other so as to allow an orientation of the surgical bed (4) to be adjusted relative to the remote center of motion;
a vertical assembly (2) having a support end (200) which is located above the surgical bed (4) and coupled to a proximal end of the setup arm (31), the vertical assembly (2) comprising a vertical translational joint (21) configured to adjust a height of the remote center of motion relative to the base (1); and
a support structure having an upper end rotatably connected to the support end (200) of the vertical assembly (2) and a lower end connected to the setup arm (31);
wherein the surgical bed (4) is connected to the base (1), and a base end (201) of the vertical assembly (2) is fixedly connected to the base (1) or the surgical bed (4); or wherein the base (1) is in an adjustable positional relationship relative to the surgical bed (4), and a based end (201) of the vertical assembly (2) is fixedly connected to the base (1); and
wherein the setup arm (31) comprises a first horizontal translational joint (311), a first rotary joint (314), a swinging joint (312) and a second rotary joint (313), which are sequentially connected together;
wherein the first horizontal translational joint (311) has an axis of translation that is perpendicular to an axis of rotation of the first rotary joint (314),
the swinging joint (312) has an axis of rotation that is perpendicular to both the axis of translation of the first horizontal translational joint (311) and the axis of rotation of the first rotary joint (314),
the second rotary joint (313) has an axis of rotation oriented parallel to the axis of rotation of the first rotary joint (314),
the first horizontal translational joint (311) connects the setup arm (31) to the support structure, the second rotary joint (313) rotatably connects the setup arm (31) to the tool arm (32).

2. The surgical robot terminal according to claim 1, wherein the axes of rotation of the first, second and third rotatable joints (410, 411, 420) intersect one another at a single point.

3. The surgical robot terminal according to claim 1 or 2, wherein the vertical assembly (2) is located on one side of the surgical bed (4).

4. The surgical robot terminal according to claim 1 or 2, wherein when the surgical bed (4) is connected to the base (1), the surgical bed (4) further comprises a bed body (41) and a support (42) that is connected to the bed body (41) and fixedly connected to the base (1), and
wherein the first, second and third rotatable joints (410, 411, 420) are all arranged on the bed body (41).

5. The surgical robot terminal according to claim 4, wherein the bed body (41) comprises an outer frame and a hook joint disposed inside and connected to the outer frame, wherein the first and second rotatable joints (410, 411) are both arranged on the hook joint, and wherein the outer frame is connected to the support (42) via the third rotatable joint (420).

6. The surgical robot terminal according to claim 4, wherein the bed body (41) comprises an outer frame and a hook joint disposed inside and connected to the outer frame, wherein the first, second and third rotatable joints (410, 411, 420) are all arranged on the hook joint, and wherein the outer frame is fixedly connected to both the vertical assembly (2) and the support (42).

7. The surgical robot terminal according to claim 1 or 2, wherein the support structure comprises
a primary arm support (9) and at least two secondary arm support (10);
wherein the primary arm support (9) has an upper end rotatably connected to the support end (200) of the vertical assembly (2); and
each of the at least two secondary arm support (10) is rotatably connected to a lower end of the primary arm support (9) and has a lower end connected to the setup arm (31).

8. The surgical robot terminal according to claim 1 or 2, wherein the support structure comprises:
at least one arm support (8), each having an upper end rotatably connected to the support end (200) of the vertical assembly (2) and a lower end connected to the setup arm (31).

9. The surgical robot terminal according to claim 1, wherein the swinging joint (312) comprises a rotatable first parallelogram structure comprising a first proximal link and a first distal link parallel to the first proximal link, the first proximal link has an axis that is parallel to or collinear with the axis of rotation of the first rotary joint (314), the first distal link is connected to the second rotary joint (313), the first distal link has an axis that is parallel to or collinear with the axis of rotation of the second rotary joint (313); or
wherein the setup arm (31) further comprises a connecting rod rotatably connected to both the swinging joint (312) and the second rotary joint (313), a measurement component configured to measure a swing angle of the swinging joint (312) and a motor configured to drive the second rotary joint (313) to swing relative to the connecting rod; wherein the measurement component is communicatively connected to the motor configured to drive the second rotary joint (313) to swing in accordance with the swing angle of the swinging joint (312) received from the measurement component so that the axis of rotation of the second rotary joint (313) is maintained parallel to the axis of rotation of the first rotary joint (314).

10. The surgical robot terminal according to claim 1 or 2, wherein tool arm (32) comprises a base joint (321), a rotatable second parallelogram structure and a telescopic joint (323);
wherein the base joint (321) is configured to pivot about a first axis so as to drive the surgical instrument to pivot about the first axis, the base joint (321) has a proximal end connected to the setup arm (31) and a distal end connected to the second parallelogram structure,
the second parallelogram structure comprising a second proximal link and a second distal link parallel to the second proximal link, the second parallelogram structure is configured to drive the surgical instrument to swing about a second axis,
the telescopic joint (323) connected to the second distal link, the telescopic joint (323) has an axis of translation that is parallel to an axis of the second distal link, the telescopic joint (323) is detachably connected to the surgical instrument so as to be able to drive the surgical instrument to translate along the axis of translation of the telescopic joint (323), and
the remote center of motion is defined at an intersection of the first axis, the second axis and the axis of translation of the telescopic joint (323); or
wherein the tool arm (32) comprises a base joint (321) and a rotatable third parallelogram structure;
wherein the base joint (321) is configured to pivot about a first axis so as to drive the surgical instrument to pivot about the first axis, the base joint (321) has a proximal end connected to the setup arm (31) and a distal end connected to the third parallelogram structure,
the third parallelogram structure comprises a third proximal link and a third distal link parallel to the third proximal link, the third parallelogram structure having a terminal end connected to the surgical instrument having an axis parallel to an axis of the distal link so that the third parallelogram structure is able to drive the surgical instrument to pivot about a second axis, and
the remote center of motion is defined at an intersection of the first axis and the second axis.

11. The surgical robot terminal according to claim 1 or 2, wherein the vertical assembly (2) further comprises a horizontal translational joint (22) that is disposed proximally at the support end (200) of the vertical assembly (2) and configured to adjust a position of the remote center of motion along a translation direction of the horizontal translational joint (22).

12. The surgical robot terminal according to claim 1, when the base (1) is in an adjustable positional relationship relative to the surgical bed (4), the surgical robot further comprises:
a plurality of universal wheel (7) disposed at a bottom of the base (1) in order to change a position of the base (1) relative to the surgical bed (4).

## Patentansprüche

1. Chirurgisches Roboterterminal, umfassend:
eine Basis (1);
mindestens einen Roboterarm (3), der jeweils einen Aufbauarm (31) und einen Werkzeugarm (32) umfasst, wobei der Werkzeugarm (32) mit einem chirurgischen Instrument verbunden ist und ausgebildet ist, das chirurgische Instrument zum Schwenken um einen entfernten Bewegungspunkt anzutreiben, wobei der Aufbauarm (31) ein distales Ende aufweist, das mit dem Werkzeugarm (32) verbunden ist, wobei der Aufbauarm (31) ausgebildet ist, eine räumliche Position des entfernten Bewegungszentrums anzupassen;
ein chirurgisches Bett (4), das ein erstes drehbares Gelenk (410), ein zweites drehbares Gelenk (411) und ein drittes drehbares Gelenk (420) umfasst, wobei die ersten, zweiten und dritten drehbaren Gelenke (410, 411, 420) jeweilige Drehachsen aufweisen, die so angeordnet sind, dass irgendzwei davon senkrecht zueinander sind, um zu erlauben, dass eine Ausrichtung des chirurgischen Betts (4) relativ zu dem entfernten Bewegungszentrum einstellbar ist;
eine vertikale Baugruppe (2) mit einem Stützende (200), das sich über dem chirurgischen Bett (4) befindet und mit einem proximalen Ende des Aufbauarms (31) gekoppelt ist, wobei die vertikale Baugruppe (2) ein vertikales Translationsgelenk (21) umfasst, das ausgebildet ist, eine Höhe des entfernten Bewegungszentrums relativ zu der Basis (1) einzustellen; und
eine Stützstruktur mit einem oberen Ende, das drehbar mit dem Stützende (200) der vertikalen Baugruppe (2) verbunden ist, und einem unteren Ende, das mit dem Aufbauarm (31) verbunden ist;
wobei das chirurgische Bett (4) mit der Basis (1) verbunden ist, und wobei ein Basisende (201) der vertikalen Baugruppe (2) fest mit der Basis (1) oder dem chirurgischen Bett (4) verbunden ist; oder wobei sich die Basis (1) in einer einstellbaren Positionsbeziehung relativ zu dem chirurgischen Bett (4) befindet, und wobei ein Basisende (201) der vertikalen Baugruppe (2) fest mit der Basis (1) verbunden ist; und
wobei der Aufbauarm (31) ein erstes horizontales Translationsgelenk (311), ein erstes Drehgelenk (314), ein Schwenkgelenk (312) und ein zweites Drehgelenk (313) umfasst, die nacheinander sequenziell verbunden sind;
wobei das erste horizontale Translationsgelenk (311) eine Translationsachse aufweist, die senkrecht zu einer Drehachse des ersten Drehgelenks (314) verläuft,
wobei das Schwenkgelenk (312) eine Drehachse aufweist, die sowohl zu der Translationsachse des ersten horizontalen Translationsgelenks (311) als auch zu der Rotationsachse des ersten Drehgelenks (314) senkrecht ist,
wobei das zweite Drehgelenk (313) eine Drehachse aufweist, die parallel zu der Drehachse des ersten Drehgelenks (314) ist,
wobei das erste horizontale Translationsgelenk (311) den Aufbauarm (31) mit der Tragstruktur verbindet, wobei das zweite Drehgelenk (313) den Aufbauarm (31) drehbar mit dem Werkzeugarm (32) verbindet.

2. Chirurgisches Roboterterminal nach Anspruch 1, wobei sich die Drehachsen der ersten, zweiten und dritten Drehgelenke (410, 411, 420) einander in einem einzigen Punkt schneiden.

3. Chirurgisches Roboterterminal nach Anspruch 1 oder 2, wobei sich die vertikale Baugruppe (2) auf einer Seite des chirurgischen Betts (4) befindet.

4. Chirurgisches Roboterterminal nach Anspruch 1 oder 2, wobei, wenn das chirurgische Bett (4) mit der Basis (1) verbunden ist, das chirurgische Bett (4) ferner einen Bettkörper (41) und eine Stütze (42) umfasst, die mit dem Bettkörper (41) verbunden und fest mit der Basis (1) verbunden ist, und
wobei die ersten, zweiten und dritten drehbaren Gelenke (410, 411, 420) alle an dem Bettkörper (41) angeordnet sind.

5. Chirurgisches Roboterterminal nach Anspruch 4, wobei der Bettkörper (41) einen Außenrahmen und ein im Inneren angeordnetes und mit dem Außenrahmen verbundenes Hakengelenk umfasst, wobei sowohl das erste als auch das zweite drehbare Gelenk (410, 411) an dem Hakengelenk angeordnet ist, und wobei der Außenrahmen über das dritte drehbare Gelenk (420) mit der Stütze (42) verbunden ist.

6. Chirurgisches Roboterterminal nach Anspruch 4, wobei der Bettkörper (41) einen Außenrahmen und ein im Inneren angeordnetes und mit dem Außenrahmen verbundenes Hakengelenk aufweist, wobei das erste, zweite und dritte Drehgelenk (410, 411, 420) alle an dem Hakengelenk angeordnet sind, und wobei der Außenrahmen sowohl mit der vertikalen Baugruppe (2) als auch mit der Stütze (42) fest verbunden ist.

7. Chirurgisches Roboterterminal nach Anspruch 1 oder 2, wobei die Stützstruktur umfasst:
eine primäre Armstütze (9) und mindestens zwei sekundäre Armstützen (10);
wobei die primäre Armstütze (9) ein oberes Ende aufweist, das drehbar mit dem Stützende (200) der vertikalen Baugruppe (2) verbunden ist; und
wobei jede der mindestens zwei sekundären Armstützen (10) drehbar mit einem unteren Ende der primären Armstütze (9) verbunden ist und ein unteres Ende aufweist, das mit dem Aufbauarm (31) verbunden ist.

8. Chirurgisches Roboterterminal nach Anspruch 1 oder 2, wobei die Stützstruktur umfasst:
mindestens eine Armstütze (8), die jeweils ein oberes Ende, das drehbar mit dem Stützende (200) der vertikalen Baugruppe (2) verbunden ist, und ein unteres Ende aufweist, das mit dem Aufbauarm (31) verbunden ist.

9. Chirurgisches Roboterterminal nach Anspruch 1, wobei das Schwenkgelenk (312) eine drehbare erste Parallelogrammstruktur umfasst, die ein erstes proximales Glied und ein zu dem ersten proximalen Glied paralleles erstes distales Glied umfasst, wobei das erste proximale Glied eine Achse aufweist, die parallel oder kollinear mit der Drehachse des ersten Drehgelenks (314) ist, wobei das erste distale Glied mit dem zweiten Drehgelenk (313) verbunden ist, wobei das erste distale Glied eine Achse aufweist, die parallel oder kollinear mit der Drehachse des zweiten Drehgelenks (313) ist; oder
wobei der Aufbauarm (31) ferner eine Verbindungsstange, die sowohl mit dem Schwenkgelenk (312) als auch dem zweiten Drehgelenk (313) drehbar verbunden ist, eine Messkomponente, die ausgebildet ist, einen Schwenkwinkel des Schwenkgelenks (312) zu messen, und einen Motor umfasst, der ausgebildet ist, das zweite Drehgelenk (313) zum Schwenken relativ zu der Verbindungsstange anzutreiben; wobei die Messkomponente kommunikativ mit dem Motor verbunden ist, der ausgebildet ist, das zweite Drehgelenk (313) zum Schwenken entsprechend dem von der Messkomponente empfangenen Schwenkwinkel des Schwenkgelenks (312) anzutreiben, so dass die Drehachse des zweiten Drehgelenks (313) parallel zu der Drehachse des ersten Drehgelenks (314) gehalten wird.

10. Chirurgisches Roboterterminal nach Anspruch 1 oder 2, wobei der Werkzeugarm (32) ein Basisgelenk (321), eine drehbare zweite Parallelogrammstruktur und ein Teleskopgelenk (323) umfasst;
wobei das Basisgelenk (321) ausgebildet ist, um eine erste Achse zu schwenken, um das chirurgische Instrument zum Schwenken um die erste Achse anzutreiben, wobei das Basisgelenk (321) ein proximales Ende, das mit dem Aufbauarm (31) verbunden ist, und ein distales Ende aufweist, das mit der zweiten Parallelogrammstruktur verbunden ist,
wobei die zweite Parallelogrammstruktur ein zweites proximales Glied und ein zu dem zweiten proximalen Glied paralleles zweites distales Glied umfasst, wobei die zweite Parallelogrammstruktur ausgebildet ist, das chirurgische Instrument zum Schwenken um eine zweite Achse anzutreiben,
wobei das Teleskopgelenk (323) mit der zweiten distalen Verbindung verbunden ist, wobei das Teleskopgelenk (323) eine Translationsachse aufweist, die parallel zu einer Achse der zweiten distalen Verbindung ist, wobei das Teleskopgelenk (323) lösbar mit dem chirurgischen Instrument verbunden ist, um in der Lage zu sein, das chirurgische Instrument entlang der Translationsachse des Teleskopgelenks (323) zum Translatieren anzutreiben, und
wobei das entfernte Bewegungszentrum an einem Schnittpunkt der ersten Achse, der zweiten Achse und der Translationsachse des Teleskopgelenks (323) definiert ist; oder
wobei der Werkzeugarm (32) ein Basisgelenk (321) und eine drehbare dritte Parallelogrammstruktur umfasst;
wobei das Basisgelenk (321) ausgebildet ist, um eine erste Achse zu schwenken, um das chirurgische Instrument zum Schwenken um die erste Achse anzutreiben, wobei das Basisgelenk (321) ein proximales Ende, das mit dem Aufbauarm (31) verbunden ist, und ein distales Ende aufweist, das mit der dritten Parallelogrammstruktur verbunden ist,
wobei die dritte Parallelogrammstruktur ein drittes proximales Glied und ein zu dem dritten proximalen Glied paralleles drittes distales Glied aufweist, wobei die dritte Parallelogrammstruktur ein mit dem chirurgischen Instrument verbundenes Anschlussende aufweist, das eine Achse aufweist, die parallel zu einer Achse des distalen Glieds ist, so dass die dritte Parallelogrammstruktur in der Lage ist, das chirurgische Instrument zum Schwenken um eine zweite Achse anzutreiben, und
wobei das entfernte Bewegungszentrum an einem Schnittpunkt der ersten Achse und der zweiten Achse definiert ist.

11. Chirurgisches Roboterterminal nach Anspruch 1 oder 2, wobei die vertikale Baugruppe (2) ferner ein horizontales Translationsgelenk (22) umfasst, das proximal an dem Stützende (200) der vertikalen Baugruppe (2) angeordnet ist und zum Einstellen einer Position des entfernten Bewegungszentrums entlang einer Translationsrichtung des horizontalen Translationsgelenks (22) ausgebildet ist.

12. Chirurgisches Roboterterminal nach Anspruch 1, wobei, wenn sich die Basis (1) in einer einstellbaren Positionsbeziehung relativ zu dem chirurgischen Bett (4) befindet, der chirurgische Roboter ferner umfasst:
eine Vielzahl von Universalrädern (7), die an der Unterseite der Basis (1) angeordnet sind, um eine Position der Basis (1) relativ zu dem chirurgischen Bett (4) zu ändern.

## Revendications

1. Un terminal de robot chirurgical, comprenant:
un socle (1);
au moins un bras robotique (3), comprenant chacun un bras de réglage (31) et un bras d'outil (32), le bras d'outil (32) étant relié à un instrument chirurgical et configuré pour entraîner l'instrument chirurgical à pivoter autour d'un centre de mouvement distant, le bras de réglage (31) ayant une extrémité distale reliée au bras d'outil (32), le bras de réglage (31) étant configuré pour ajuster une position spatiale du centre de mouvement distant;
un lit chirurgical (4) comprenant une première articulation rotative (410), une deuxième articulation rotative (411) et une troisième articulation rotative (420), les première, deuxième et troisième articulations rotatives (410, 411, 420) ayant des axes de rotation respectifs agencés de sorte que deux quelconques d' entre eux soient perpendiculaires l'un à l'autre de manière à permettre à une orientation du lit chirurgical (4) d'être ajustée par rapport au centre de mouvement distant;
un ensemble vertical (2) ayant une extrémité de support (200) qui est située au-dessus du lit chirurgical (4) et couplée à une extrémité proximale du bras de montage (31), l'ensemble vertical (2) comprenant une articulation de translation verticale (21) configuré pour ajuster une hauteur du centre de mouvement distant par rapport à la base (1); et
une structure de support ayant une extrémité supérieure reliée de manière rotative à l'extrémité de support (200) de l'ensemble vertical (2) et une extrémité inférieure reliée au bras de montage (31);
dans lequel le lit chirurgical (4) est relié à la base (1), et une extrémité de base (201) de l'ensemble vertical (2) est reliée de manière fixe à la base (1) ou au lit chirurgical (4); ou dans lequel la base (1) est dans une relation de position réglable par rapport au lit chirurgical (4), et une extrémité de base (201) de l'ensemble vertical (2) est reliée de manière fixe à la base (1); et
dans lequel le bras de réglage (31) comprend une première articulation de translation horizontale (311), une première articulation rotative (314), une articulation oscillante (312) et une seconde articulation rotative (313), qui sont séquentiellement reliées ensemble;
dans lequel la première articulation de translation horizontale (311) a un axe de translation qui est perpendiculaire à un axe de rotation de la première articulation rotative (314),
l'articulation oscillante (312) présente un axe de rotation perpendiculaire à la fois à l'axe de translation de la première articulation de translation horizontale (311) et à l'axe de rotation de la première articulation rotative (314),
le second joint tournant (313) a un axe de rotation orienté parallèlement à l'axe de rotation du premier joint tournant (314),
la première articulation de translation horizontale (311) relie le bras de réglage (31) à la structure de support, le deuxième joint tournant (313) relie de manière rotative le bras de réglage (31) au bras d'outil (32).

2. Terminal de robot chirurgical selon la revendication 1, dans lequel les axes de rotation des première, deuxième et troisième articulations rotatives (410, 411, 420) se coupent en un point unique.

3. Terminal de robot chirurgical selon la revendication 1 ou 2, dans lequel l'ensemble vertical (2) est situé d'un côté du lit chirurgical (4).

4. Terminal de robot chirurgical selon la revendication 1 ou 2, dans lequel lorsque le lit chirurgical (4) est relié à la base (1), le lit chirurgical (4) comprend en outre un corps de lit (41) et un support (42) qui est relié au corps de lit (41) et relié de manière fixe à la base (1), et dans lequel les premier, deuxième et troisième joints rotatifs (410, 411, 420) sont tous agencés sur le corps de lit (41).

5. Terminal de robot chirurgical selon la revendication 4, dans lequel le corps de lit (41) comprend un cadre externe et un joint à crochet disposé à l'intérieur et relié au cadre externe, dans lequel les premier et second joints rotatifs (410, 411) sont tous deux agencé sur l'articulation à crochet, et dans lequel le cadre extérieur est relié au support (42) via la troisième articulation rotative (420).

6. Terminal de robot chirurgical selon la revendication 4, dans lequel le corps de lit (41) comprend un cadre extérieur et une articulation à crochet disposée à l'intérieur et reliée au cadre extérieur, dans lequel les première, deuxième et troisième articulations rotatives (410, 411, 420) sont tous agencés sur l'articulation à crochet, et dans lequel le cadre extérieur est relié de manière fixe à la fois à l'ensemble vertical (2) et au support (42).

7. Terminal de robot chirurgical selon la revendication 1 ou 2, dans lequel la structure de support comprend:
un support de bras principal (9) et au moins deux supports de bras secondaires (10);
dans lequel le support de bras principal (9) a une extrémité supérieure reliée de manière rotative à l'extrémité de support (200) de l'ensemble vertical (2); et
chacun des au moins deux supports de bras secondaires (10) est relié de manière rotative à une extrémité inférieure du support de bras principal (9) et a s une extrémité inférieure reliée au bras de montage (31).

8. Terminal de robot chirurgical selon la revendication 1 ou 2, dans lequel la structure de support comprend:
au moins un support de bras (8), chacun ayant une extrémité supérieure reliée de manière rotative à l'extrémité de support (200) de l'ensemble vertical (2) et une extrémité inférieure reliée au bras de réglage (31).

9. Terminal de robot chirurgical selon la revendication 1, dans lequel l'articulation oscillante (312) comprend une première structure de parallélogramme rotatif comprenant une première liaison proximale et une première liaison distale parallèle à la première liaison proximale, la première liaison proximale a un axe qui est parallèle. à ou colinéaire à l'axe de rotation de la première articulation rotative (314), la première liaison distale est relié au second joint tournant (313), le premier lien distal a un axe parallèle ou colinéaire à l'axe de rotation du second joint tournant (313); ou
dans lequel le bras de réglage (31) comprend en outre une bielle reliée de manière rotative à la fois au joint oscillant (312) et au second joint rotatif (313), un composant de mesure configuré pour mesurer un angle d'oscillation du joint oscillant (312) et un moteur configuré pour entraîner le deuxième joint rotatif (313) en oscillation par rapport à la bielle; dans lequel le composant de mesure est connecté en communication au moteur configuré pour entraîner le deuxième joint rotatif (313) pour qu'il oscille conformément à l'angle d'oscillation du joint oscillant (312) reçu du composant de mesure de sorte que l'axe de rotation du second joint tournant (313) soit maintenu parallèle à l'axe de rotation du premier joint tournant (314).

10. Terminal de robot chirurgical selon la revendication 1 ou 2, dans lequel le bras d'outil (32) comprend une articulation de base (321), une seconde structure de parallélogramme rotative et une articulation télescopique (323);
dans lequel l'articulation de base (321) est configurée pour pivoter autour d'un premier axe de manière à entraîner l'instrument chirurgical pour pivoter autour du premier axe, l'articulation de base (321) a une extrémité proximale reliée au bras de montage (31) et un extrémité distale reliée à la deuxième structure en parallélogramme,
la seconde structure en parallélogramme comprenant une seconde liaison proximale et une seconde liaison distale parallèle à la seconde liaison proximale, la seconde structure en parallélogramme est configurée pour entraîner l'instrument chirurgical à osciller autour d'un second axe,
l'articulation télescopique (323) reliée au deuxième lien distal, l'articulation télescopique (323) a un axe de translation parallèle à un axe du deuxième lien distal, l'articulation télescopique (323) est reliée de manière amovible à l'instrument chirurgical de manière à pouvoir entraîner l'instrument chirurgical en translation le long de l'axe de translation de l'articulation télescopique (323), et
le centre de mouvement distant est défini à une intersection du premier axe, du deuxième axe et de l'axe de translation de l'articulation télescopique (323); ou
dans lequel le bras d'outil (32) comprend une articulation de base (321) et une troisième structure de parallélogramme rotative;
dans lequel l'articulation de base (321) est configurée pour pivoter autour d'un premier axe de manière à entraîner l'instrument chirurgical pour pivoter autour du premier axe, l'articulation de base (321) a une extrémité proximale reliée au bras de montage (31) et un extrémité distale reliée à la troisième structure en parallélogramme,
la troisième structure en parallélogramme comprend un troisième lien proximal et un troisième lien distal parallèle au troisième lien proximal, la troisième structure en parallélogramme ayant une extrémité terminale reliée à l'instrument chirurgical ayant un axe parallèle à un axe du lien distal de sorte que le troisième la structure en parallélogramme est capable d'entraîner l'instrument chirurgical à pivoter autour d'un second axe, et
le centre de mouvement éloigné est défini à une intersection du premier axe et du deuxième axe.

11. Terminal de robot chirurgical selon la revendication 1 ou 2, dans lequel l'ensemble vertical (2) comprend en outre une articulation de translation horizontale (22) qui est disposée de manière proximale à l'extrémité de support (200) de l'ensemble vertical (2) et configurée pour ajuster un position du centre de mouvement distant le long d'une direction de translation de l'articulation de translation horizontale (22).

12. Terminal de robot chirurgical selon la revendication 1, lorsque la base (1) est dans une relation de position réglable par rapport au lit chirurgical (4), le robot chirurgical comprend en outre:
une pluralité de roues universelles (7) disposées au fond de la base (1) afin de modifier une position de la base (1) par rapport au lit chirurgical (4).
